# EUROPEAN PATENT SPECIFICATION

(11) **EP 4 135 723 B1**
(45) Date of publication and mention of the grant of the patent: **12.03.2025**
(21) Application number: 20720401.7
(22) Date of filing: 16.04.2020
(51) Int. Cl.: A61K 33/38, A61K 31/245, A61K 31/255, A61K 31/4184, A61K 31/426, A61P 31/04, A61P 31/12

(54) **ANTIMICROBIAL SILVER COORDINATION COMPLEXES**
ANTIMICROBISCHE SILBER KOORDINATION COMPLEXE
COMPLEXES ANTIMICROBIENS DE COORDINATION AVEC ARGENT

(43) Date of publication of application: 22.02.2023
(73) Proprietor: DEBx Medical Holding B.V., 1083 HN Amsterdam (NL)
(72) Inventor: BIGNOZZI, Carlo, Alberto, 44121 Ferrara (IT); QUINT, Bertus, Jozef, 1082 MA Amsterdam (NL); COGO, Alberto, 36100 Vicenza (IT)
(74) Representative: Nederlandsch Octrooibureau
(86) International application number: PCT/EP2020/060686
(87) International publication number: WO 2021/209134

(56) References cited:
- WO-A1-2019/135136
- US-B2- 8 124 826
- PROSPOSITO PAOLO ET AL: "Hydrophilic silver nanoparticles with tunable optical properties: application for the detection of heavy metals in water", vol. 7, 1 January 2016 (2016-01-01), pages 1654 - 1661, XP009524908, ISSN: 2190-4286, Retrieved from the Internet <URL:https://www.beilstein-journals.org/bjnano/content/pdf/2190-4286-7-157.pdf> [retrieved on 20161017], DOI: 10.3762/BJNANO.7.157
- PORCARO FRANCESCO ET AL: "Synthesis and Structural Characterization of Silver Nanoparticles Stabilized with 3-Mercapto-1-Propansulfonate and 1-Thioglucose Mixed Thiols for Antibacterial Applications", vol. 9, no. 12, 20 December 2016 (2016-12-20), pages 1028, XP009524907, ISSN: 1996-1944, Retrieved from the Internet <URL:http://www.mdpi.com/1996-1944/9/12/1028> [retrieved on 20161220], DOI: 10.3390/MA9121028
- SHERINE JOSITTA ET AL: "Ag(I) and Au(III) Mercaptobenzothiazolecomplexes induced apoptotic cell death", vol. 9, no. 1, 24 January 2019 (2019-01-24), pages ArticleNo, XP009524904, ISSN: 2045-2322, Retrieved from the Internet <URL:https://www.ncbi.nlm.nih.gov/pmc/?term=%22Sci+Rep%22 journal!> DOI: 10.1038/S41598-018-36801-6

## Description

### Field of the invention

The invention relates to coordination complexes that are provided with antimicrobial activity. The aforesaid coordination complexes can be incorporated in hyaluronic acid compositions and can form salts with surfactants. The hyaluronic acid compositions incorporating the aforesaid coordination complexes and the salts formed by the aforesaid coordination complexes are suitable for medical use, in particular for topical treatments.

### Prior Art

Hyaluronic acid (HA) is a linear polysaccharide composed of repeating disaccharide units, which in turn consist of glucuronic acid and N-acetyl glucosamine. Hyaluronic acid is commonly used in the form of physiologically and pharmaceutically acceptable salt, in particular in the form of sodium salt (sodium hyaluronate).

Hyaluronic acid is one of the major matrix substances in which cells and fibrous constituents of the matrix, such as collagen and elastin, are embedded. Another unique property of hyaluronic acid is the enormously high water binding capacity thereof. In water solutions, hyaluronic acid has a flexible and spiralized configuration, which configuration is able to contain approximately 1000-fold more water than the polymer as sampled (Biopolymers 1970; 9:799-810). This special property enables hyaluronic acid to contribute largely to the maintenance of the extracellular space and to control tissue hydration.

Since the discovery thereof (J. Biol. Chem. 1934, 107, 629-634), hyaluronic acid has received great attention as a versatile and highly functional biopolymer (BioDrugs 2012, 26, 257-268, Carbohydr. Polym. 2013, 92, 1262- 1279.2-6). Clinically, hyaluronic acid has been used in several applications, including ophthalmology as a drug delivery system (Optom. Vis. Sci. 2014, 91, 32-38), in orthopaedics for osteoarthritis treatments (i.e. visco-supplementation) (Eur. Rev. Med. Pharmacol. Sci. 2014, 18, 3326-3338) and as a dermal filler (Facial Plast. Surg. 2014, 30, 81-83,16,17).

Hyaluronic acid has a number of roles in the initial inflammatory stages, such as providing a structural framework via the interaction with the fibrin clot, which modulates the cellular inflammatory infiltration of the extracellular matrix in the inflammatory site. Moreover, hyaluronic acid has been identified in all periodontal tissues, being particularly prominent in the non-mineralized tissues, such as gingiva and periodontal ligament, and being contained in only low quantities in mineralized tissues, such as cementum and alveolar bone. It is known that periodontal tissues can undergo periodontal diseases, which are caused by bacterial infections inducing inflammatory responses with progressive destruction of the periodontal tissues and finally the lost of teeth.

The inclusion of silver complexes (silver coordination compounds) in a hyaluronic acid matrix enables antimicrobial formulations to be obtained that are particularly useful for several applications, e.g. for the treatment of periodontal disease. In fact, the activity of silver as antimicrobial agent has been well established in past years (Prog. Med. Chem. 1994). The mechanism of the antimicrobial action of silver ions is closely related to the interaction thereof with thiol groups that are comprised in enzymes and proteins, although other cellular components may be involved. Virucidal properties of silver ions might also be explained in view of the capacity thereof of binding to SH groups (Crit. Rev. Environ. Control 1989, 18:295-315.), as well as through the preferential interaction thereof with the bases in DNA (Chem. Rev. 1971, 71:439-471).

A significant drawback of known silver-based compositions, however, is that they suffer very often from thermal and photochemical instability, as well as from low solubility.

In addition, although anionic silver complexes can be compatible with aqueous solutions of sodium hyaluronate, the thermal stability of these antimicrobial preparations is still an issue to be solved. Document WO 2019/135136 discloses the antimicrobial activity of silverhyaluronate gel containing a mono-coordinated complex Na⁺[Ag⁺(L²⁻)] or a complex Na⁺₂ [Ag⁺(2-mercaptobenzoate²⁻) (2-hydroxybenzoate²⁻)], which are both different from the claimed silver complex according to formula [Ag⁺(L²⁻)₂]³⁻.

Therefore, a strong need is felt for antimicrobial silver-based compositions that are not affected by the above-mentioned drawbacks, such as thermal and photochemical instability, low solubility. In particular, a strong need is felt for antimicrobial silver-based compositions, in which silver ions are incorporated within a hyaluronic acid matrix and which are thermally stable.

### Objects of the invention

An object of the invention is to improve known antimicrobial silver-based compositions.

Another object is to improve known antimicrobial compositions, in which silver ions are incorporated in a hyaluronic acid matrix.

Another object is to make available antimicrobial compositions that contain silver ions and are provided with suitable thermal and photochemical stability and high solubility.

Yet another object is to make available antimicrobial compositions that contain silver ions, are thermally and photochemically stable and high soluble and can be effectively used in topical therapeutic treatments.

### Brief description of the invention

According to the invention, an anionic bi-coordinated silver (I) complex is provided having antimicrobial activity, as defined in claim 1.

The invention enables the drawbacks of known antimicrobial silver-based compositions, and in particular of antimicrobial silver-containing hyaluronic acid compositions, to be overcome. In fact, owing to the invention, anionic silver complexes are provided, which have a negative charge of -3 and in which the silver ion (Ag⁺) is bound to two anionic ligands. The Applicant found and experimentally verified that the complexes according to the invention possess a remarkable antimicrobial activity. The aforesaid complexes enable an antimicrobial (transparent and odourless) gel to be prepared in association with sodium hyaluronate and sodium ascorbyl phosphate (stable form of C vitamin). The aforesaid antimicrobial gel exhibits unprecedented thermal and photochemical stability and is suitable for medical use. In particular, it may be used as a liquid bandage for topical use, which is suitable for protecting skin or gingival wounds from microbial attack. The antimicrobial gel has also shown antifungal properties, owing to which it can be used in the gynaecological field. Applications can be foreseen in the dental field

In addition, the Applicant found and experimentally verified that, owing to the above-mentioned negative charge (3-), the anionic silver complexes can be associated to cationic compounds that are surface-active agents and disinfectants, such as benzalkonium, didecyldimethylammonium, chlorhexidine and octenidine, so as to form salts. These complex-containing salts are insoluble in water, but very soluble in alcoholic solvents, such as methanol, ethanol, n-propanol and (to a lesser degree) 2-propanol. The complex-containing salts show a very high affinity for any kind of solid surface and can be adsorbed e.g. on silica particles (functionalized silica particles), thus enabling antimicrobial powders and solutions to be formulated that are suitable for medical use, namely for topical treatments.

### Brief description of the drawings

The invention can be better understood and implemented with reference to the enclosed drawings that illustrate an embodiment thereof by way of example in which:

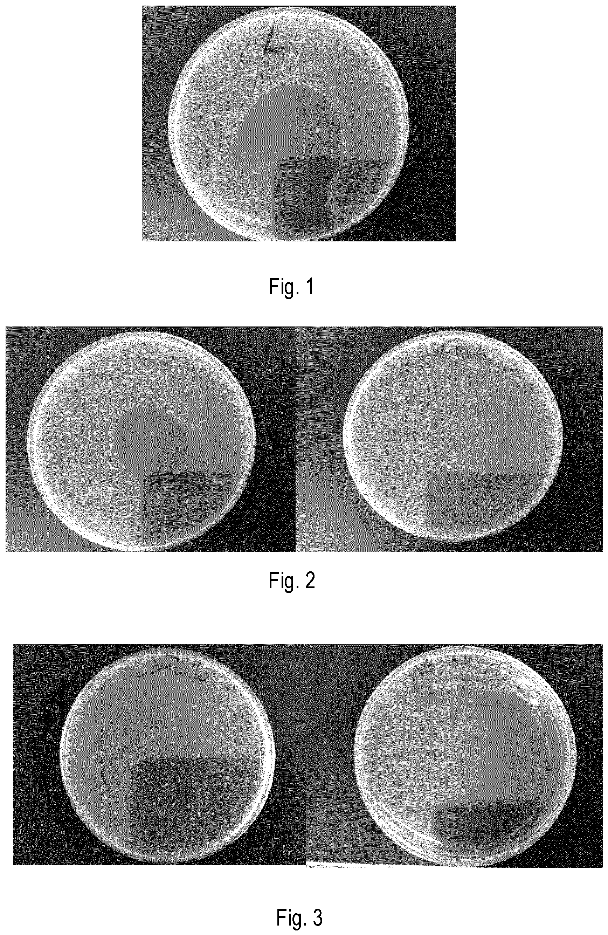

Figure 1 is a photograph of a Petri dish, showing a microbial growth inhibition halo observed after depositing of a sample of an aqueous solution comprising the anionic silver complex according to the invention in the form of a sodium salt;
Figure 2 is a photograph of two Petri dishes, showing microbial growth inhibition halos observed after depositing of a sample of a hyaluronate gel containing the anionic silver complex according to the invention in the form of a sodium salt (Petri dish on the left) and after depositing a sample of a control gel (Petri dish on the right);
Figure 3 is a photograph of two Petri dishes, showing a different microbial proliferation after contact (Petri dish on the right) and without contact (Petri dish on the left) with the anionic silver complex according to the invention in the form of a benzalkonium salt;

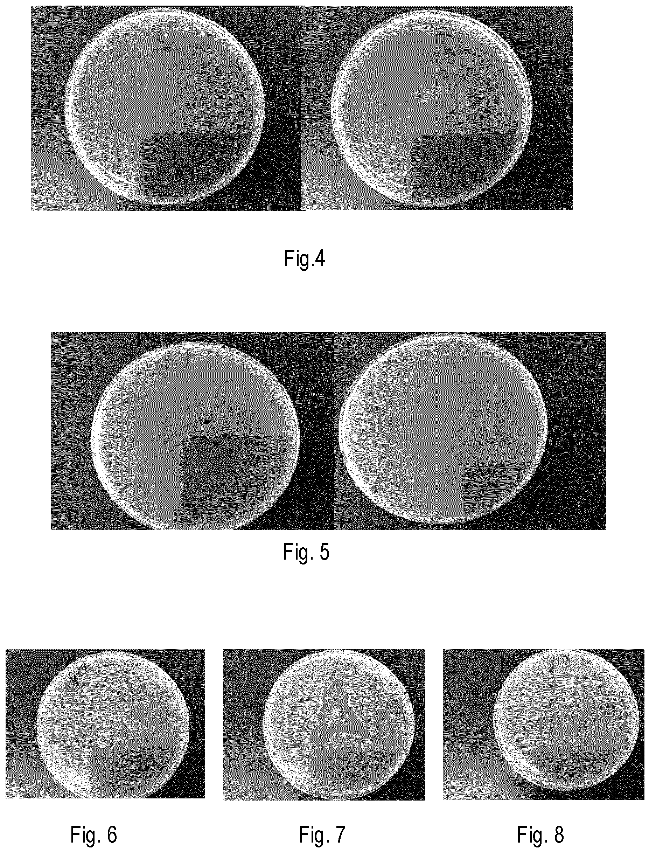

Figure 4 is a photograph of two Petri dishes, showing a comparison between a microbial proliferation in the presence of the anionic silver complex according to the invention in the form of a didecyldimethylammonium salt (Petri dish on the left) and a microbial proliferation in the presence of the anionic silver complex according to the invention in the form of an octenidine salt (Petri dish on the right);
Figure 5 is a photograph of two Petri dishes, showing a comparison between a microbial proliferation in the presence of the anionic silver complex according to the invention in the form of a benzalkonium salt (Petri dish on the left) and a microbial proliferation in the presence of the anionic silver complex according to the invention in the form of an didecyldimethylammonium salt (Petri dish on the right);
Figures 6 to 8 are three photographs of Petri dishes, showing microbial growth inhibition halos observed after depositing of silica particles, which silica particles have been functionalized with the anionic silver complex according to the invention in the form of an octenidine salt (Figure 6), a chlorhexidine salt (Figure 7) and a benzalkonium salt (Figure 8);

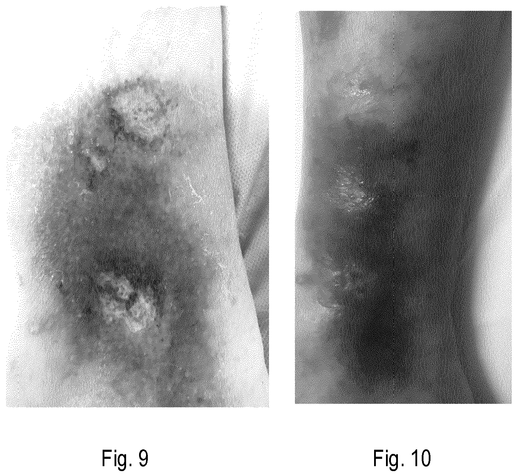

Figure 9 is a photograph, showing a chronic skin lesion before a topical treatment through with a composition containing the anionic silver complex according to the invention;
Figure 10 is a photograph, showing the same chronic skin lesion of Figure 9 after a three week-period of topical treatment with the composition containing the anionic silver complex according to the invention.

Detailed description of the invention

In the present description, as well as in the appended claims:
- the term "silver ion" and the corresponding chemical symbols "Ag⁺" or "Ag(I)" are used interchangeably;
- the terms "complex(es)", "coordination complex(es)" and "coordination compound(s)" are considered to be synonyms and can be used interchangeably;
- the terms "microorganism(s)" and "microbe(s)" are considered to be synonyms and can be used interchangeably;
- the term "antimicrobial" defines the capacity of a substance, or mixture of substances, to kill microorganisms in general and/or to prevent the development thereof;
- the term "antibacterial" defines the capacity of a substance, or mixture of substances, to kill specifically bacteria (bactericide action) and/or to prevent specifically the development of bacteria (bacteriostatic action).

The anionic bi-coordinated silver complex according to the invention has the following general formula:

[Ag⁺(L²⁻)(L²⁻)]³⁻ or [Ag⁺(L²⁻)₂]³⁻

As it can be seen from the general formula above, the silver ion binds two ligands, each of which bears two negative charges. The overall charge of the complex is therefore 3- and, owing to this, a high solubility in aqueous solution is exhibited by a sodium salt formed by the complex according to the invention and having the following general formula:

Na⁺₃[Ag⁺(L²⁻)(L²⁻)]³⁻ or Na⁺₃[Ag⁺(L²⁻)₂]³⁻

The activity of silver complexes against microbial species is strictly connected with the solubility, thermal stability and photochemical stability thereof. These properties are rigidly ruled by the choice of suitable ligands (L), and by slight modulations in the steric and electronic effects thereof. The identification of such ligands and the compatibility of the charge thereof with the anionic form of hyaluronic acid would allow to prepare gels having an extraordinary antimicrobial power.

Suitable ligands identified by the Applicant are disclosed here below.

A suitable ligand is derived from 2-mercapto-4-methyl-5-thiazoleacetic acid (MW = 189.26 g/mol; CAS: 34272-64-5), hereinafter "MPA":

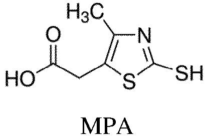

Following deprotonation of the carboxylic (-COOH) and mercapto (-SH) groups of MPA, a bivalent anionic form of 2-mercapto-4-methyl-5-thiazoleacetic acid is obtained. The bivalent anionic form of 2-mercapto-4-methyl-5-thiazoleacetic acid, hereinafter "MPA²⁻" ligand, enables a bi-coordinated anionic complex to be formed, which bi-coordinated complex has the following molecular formula:

[Ag⁺(MPA²⁻)₂]³⁻

and the following structural formula:

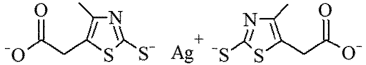

The bi-coordinated complex above can form a sodium salt, which exhibit a very high solubility in aqueous solution and has the following general formula:

Na⁺₃[Ag⁺(MPA²⁻)₂]³⁻

Another suitable ligand is derived from 3-mercapto-1-propansulfonate, hereinafter "MPS²⁻", deriving from 3-mercapto-1-propansulfonate sodium salt (MW= 178.21 g/mol; CAS17636-10-1), hereinafter "NaMPS":

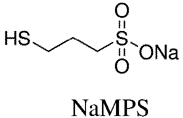

The MPS²⁻ ligand enables a bi-coordinated negative charged (3-) complex to be formed, having the following molecular formula:

[Ag⁺(MPS²⁻)₂]³⁻

and the following structural formula:

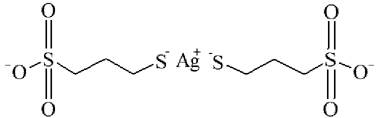

The bi-coordinated anionic complex above can form a sodium salt, which exhibit a very high solubility in aqueous solution and has the following formula:

Na⁺₃[Ag⁺(MPS²⁻)₂]³⁻

Both the above disclosed bi-coordinated anionic complexes [Ag⁺(MPA²⁻)₂]³⁻ and [Ag⁺(MPS²⁻)₂]³⁻ show a remarkable photochemical and thermal stability in aqueous solution. Moreover, both the anionic complexes are perfectly compatible with sodium hyaluronate, so that stable sodium hyaluronate gel solutions can be prepared that contain high concentration of sodium hyaluronate, namely a concentration of sodium hyaluronate in the range 0.8-1.4%. The aforesaid gel solutions are thermally stable, as it has been verified by monitoring the electronic absorption spectra and the viscosity. No changes of viscosity and/or in the electronic absorption have been observed after 40 days at 55°, which indicates a substantially optimal compatibility between the silver complexes according to the invention and a sodium hyaluronate composition. The complex-containing sodium hyaluronate gel solutions are suitable for medical use, in particular as a liquid bandage that is suitable for protecting topical or gingival wounds from microbial attack.

A further peculiar property of the complexes above - and more generally of the complex according to the invention - is that they are stable in presence of sodium-ascorbyl-phosphate (SAP), which in turn is a stable form of C vitamin and is known to act as a reducing agent and a radical scavenger. The silver ions present in the complexes are not reduced by SAP, which can thus be added to different formulations based on the silver complexes according to the invention.

A further suitable ligand is 2-mercapto-5-benzimidazole sulfonate, hereinafter "X²⁻", derived from the sodium salt of 2-mercapto-5-benzimidazole sulfonic acid, hereinafter "NaX":

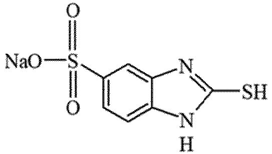

Following deprotonation of the SH moiety, the X²⁻ ligand enables a bi-coordinated negative charged (3-) complex to be formed, having the following molecular formula:

[Ag⁺(X²⁻)₂]³⁻

The bi-coordinated anionic complex above can form a sodium salt, which exhibit a very high solubility in aqueous solution, is thermally and photochemically stable and has the following formula:

Na⁺³[Ag⁺(X²⁻)₂]³⁻

A yet further suitable ligand is thiosalicylate, hereinafter "TS²⁻", derived from thiosalicylic acid:

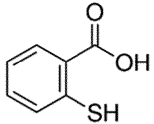

Following deprotonation of the carboxylic (-COOH) and mercapto (-SH) groups, the TS²⁻ ligand enables a bi-coordinated negative charged (3-) complex to be formed, having the following molecular formula:

[Ag⁺(TS²⁻)₂]³⁻

The bi-coordinated anionic complex above can form a sodium salt, which exhibit a very high solubility in aqueous solution, is thermally and photochemically stable and has the following formula:

Na⁺³[Ag⁺(TS²⁻)₂]³⁻

Also the bi-coordinated anionic complexes [Ag⁺(X²⁻)₂]³⁻ and [Ag⁺(TS²⁻)₂]³ are compatible with sodium hyaluronate, so that stable sodium hyaluronate gel solutions incorporating [Ag⁺(X²⁻)₂]³⁻or [Ag⁺(TS²⁻)₂]³ can be prepared.

An additional and remarkably interesting property of the anionic silver complex according to the invention is the capability thereof of forming salts (complex salts) with cationic surface-active agents. Neutral salts can be obtained by adding stoichiometric amounts of the silver complexes in the form of sodium salts - i.e. Na⁺₃[Ag⁺(MPA²⁻)₂]³⁻, Na⁺₃[Ag⁺(MPS²⁻)₂]³⁻, Na⁺³[Ag⁺(X²⁻)₂]³⁻ or Na⁺³[Ag⁺(TS²⁻)₂]³⁻ - to known antimicrobial surface-active agents that have been identified (by the Applicant) as suitable for such an use. The suitable surface-active agents include the following:

### Octenidine (hereinafter "OCT")

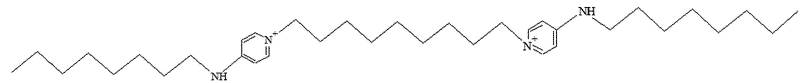

### Chlorhexidine (hereinafter "CHL")

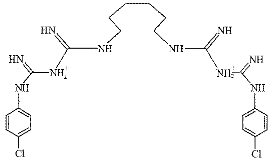

### Benzalkonium (hereinafter "BZ")

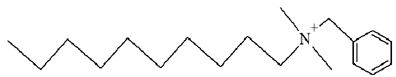

### Didecyldimethylammonium (hereinafter "DDA")

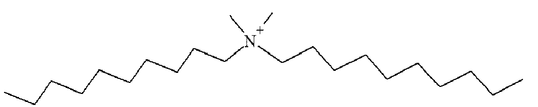

Owing to the high negative charge (3-) of the anionic silver complexes, a strong interaction takes places between anion and cations, resulting in salts which are insoluble in water but surprisingly very soluble in polar solvents, such as e.g. methanol, ethanol, n-propanol, dimethyl sulfoxide (DMSO) and also (to a lesser degree) in 2-propanol. The new salts show a very high affinity for any kind of solid surface and can be adsorbed on silica particles (functionalized silica particles), thus enabling antimicrobial powders and solutions to be formulated that are suitable for medical use, namely for topical treatments.

In view of the positive charges of the cations OCT, CHL (2+) and BZ, DDA (1+), the resulting molecular formulae of the aforesaid salts are as follows:

(OCT)₃[Ag⁺(MPA²⁻)₂]₂, (CHL)₃[Ag⁺(MPA²⁻)₂]₂, (BZ)₃[Ag⁺(MPA²⁻)₂], (DDA)₃[Ag⁺(MPA²)₂];

(OCT)₃[Ag⁺(MPS²⁻)₂]₂, (CHL)₃[Ag⁺(MPS²⁻)₂]₂, (BZ)₃[Ag⁺(MPS²⁻)₂], (DDA)₃[Ag⁺(MPS²)₂];

(OCT)₃[Ag⁺(X²⁻)₂]₂, (CHL)₃[Ag⁺(X²⁻)₂]₂, (BZ)₃[Ag⁺(X²⁻)₂]; (DDA)₃[Ag⁺(X²⁻)₂];

(OCT)₃[Ag⁺(TS²⁻)₂]₂, (CHL)₃[Ag⁺(TS²⁻)₂]₂, (BZ)₃[Ag⁺(TS²⁻)₂], (DDA)₃[Ag⁺(TS²⁻)₂].

The various salts disclosed above can be encompassed by the following general formula:

(W)₃[Ag⁺(L²⁻)₂]ₙ

wherein:
- W is a cationic surface-active agent, selected from the group consisting of octenidine, chlorhexidine, benzalkonium and didecyldimethylammonium;
- n is 2 when W is octenidine or chlorhexidine;
- n is 1 when W is benzalkonium or didecyldimethylammonium.

The aforesaid salts are insoluble in water, but they are soluble in alcoholic solvents such as e.g. methanol, ethanol, 1-propanol, 2-propanol, 3-methoxy-3-methyl-1-butanol and are also very soluble in dimethyl sulfoxide (DMSO). The salts according to the invention show a very high affinity for solid surfaces, which can be made of different materials - such as e.g. glass, metal, plastics, wood, graphite and cellulose - and onto which the salts are able to adsorb owing to Van der Waals forces.

The salts according to the invention can thus be used to formulate interesting antimicrobial solutions, e.g. antimicrobial volatile spray solutions, for surface coatings. Examples of embodiments of the aforesaid surface coatings include, but are not limited to: coatings of conduits and filters for air conditioning in buildings, cars, airplanes, clean rooms; coatings for air decontamination devices; coatings of protective masks; coatings of titanium and stainless steel surgical implants.

Moreover, the salts according to the invention can be used to produce medicaments in the form of antimicrobial powders. These powders are intended for topical use and are made of silica particles having the antimicrobial salts adsorbed thereon (functionalized silica particles). In other words, it is possible to prepare a powder comprising silica particles, onto which the salts according to the invention are adsorbed, for use in an antimicrobial topical treatment. Suitable methods and apparatuses for adsorbing salts onto solid, e.g. silica, particles are known to the person skilled in the art and thus will not be disclosed herein.

In order to better explain the invention and facilitate the understanding thereof, but without limiting the scope of the invention, the following Examples are disclosed: preparation of an aqueous solution containing Na⁺₃[Ag⁺(MPA⁻²)₂]⁻³ (Example 1); preparation of a hyaluronate gel containing Na⁺₃[Ag⁺(MPA⁻²)₂]⁻³ (Example 2); preparation of a hyaluronate gel containing Na⁺₃[Ag⁺(MPA⁻²)₂]⁻³ and sodium ascorbyl phosphate (Example 3); preparation of an aqueous solution containing Na⁺₃[Ag⁺(MPS⁻²)₂]⁻³ (Example 4); preparation of a hyaluronate gel containing Na⁺₃[Ag⁺(MPS⁻²)₂]⁻³ (Example 5); preparation of a hyaluronate gel containing Na⁺₃[Ag⁺(MPS⁻²)₂]⁻³ and sodium ascorbyl phosphate (Example 6); preparation of an aqueous solution containing Na⁺₃[Ag⁺(X⁻²)₂]⁻³ (Example 7); preparation of an aqueous solution containing Na⁺₃[Ag⁺(TS⁻²)₂]⁻³ (Example 8); preparation of salts formed by the silver complex with cationic surface-active agents (Example 9); preparation of a microbial pool for experimental use (Example 10); tests on solutions and gel containing the silver complex according to the invention (Example 11); surface tests on ethanol solutions of salts formed by the silver complex according to the invention with cationic surface-active agents (Example 12); tests on silica particles functionalized with the salts formed by the silver complex according to the invention with cationic surface-active agents (Example 13); topical treatment of a chronic skin lesion (Example 14).

### Example 1 - Preparation of an aqueous solution containing Na⁺₃[Ag⁺(MPA⁻²)₂]⁻³

Reactants: MPA = 2-mercapto-4-methyl-5-thiazolacetic acid, MW = 189.26, CAS: 34272-64-5; Ag(NO₃), MW 169.87, CAS 7761-88-8.

To a 1.135 g MPA amount, 12.36 g of a 1M NaOH aqueous solution are added. To the so obtained suspension 186.5 g of distilled water are then added and the solution is kept under stirring for 5 min. 100 g of an aqueous solution containing 0.51 g di AgNO₃ are finally added and a yellow solution is obtained, which is kept under stirring for additional 30 min. The Na⁺₃[Ag⁺(MPA⁻²)₂]⁻³ complex-containing salt is precipitated by adding an excess of acetone, filtered and air dried. %Ag⁺ = 0.108%.

Elemental analysis on the solid product gave the following results: calculated for Na₃AgC₁₂N₂S₄O₄H₁₀: C 26.14; N, 5.08; H, 1.83; Ag, 19.57. Found: C, 25.87; N 5.0; H 1. 88; Ag 19.46.

Elemental analysis of carbon, nitrogen and hydrogen was performed with a LECO CHN analyzer. Silver was determined through plasma atomic absorption spectrometry by Perkin Elmer Optima 3100 XL.

### Example 2 - Preparation of a hyaluronate gel containing Na⁺₃[Ag⁺(MPA⁻²)₂]⁻³

To a 300 g amount of the aqueous solution of Na⁺₃[Ag⁺(MPA⁻²)₂]⁻³ disclosed in Example 1, 96 g of distilled water and 4 g of sodium hyaluronate are added; the mixture is kept under gentle stirring for 12-16 h, until complete jellification. %Ag⁺ = 0.081%

### Example 3 - Preparation of a hyaluronate gel containing Na⁺₃[Ag⁺(MPA⁻²)₂]⁻³ and sodium ascorbyl phosphate

To a 300 g amount of the aqueous solution of Na⁺₃[Ag⁺(MPA⁻²)₂]⁻³ disclosed in Example 1, 20g of sodium ascorbyl phosphate are added, followed by 76 g of distilled water and 4 g of sodium hyaluronate. The mixture is kept under gentle stirring for 12-16 h, until complete jellification. %Ag⁺ = 0.081%.

### Example 4 - preparation of an aqueous solution containing Na⁺₃[Ag⁺(MPS⁻²)₂]⁻³

Reactants: NaMPS = 3-mercapto-1-propansulfonate sodium salt, MW= 178.21 g/mol, CAS: 17636-10-1; Ag(NO₃), MW 169.87, CAS 7761-88-8.

1.648 g of NaMPS are added to 9.58 g of a 1M NaOH solution. After dissolution, 250 g of distilled water are added, followed by addition of 238.8 g of an aqueous solution containing 0.79 g of AgNO₃ dissolved therein. After the addition of the silver salt the solution becomes light yellow in colour. The Na⁺₃[Ag⁺(MPS⁻²)₂]⁻³ complex-containing salt is precipitated by addition of an excess of acetone, filtered and air dried. %Ag⁺ = 0.1%.

Elemental analysis on the solid product gave the following results: calculated for Na₃AgS₄O₆C₆H₁₂: C, 14.85; S, 26.43; H, 2.49; Ag, 22.23. Found: C, 14.32; S, 26.24; H 2.58; Ag 21.90. Elemental analysis of carbon, nitrogen and hydrogen was performed with a LECO CHN analyzer. Silver was determined through plasma atomic absorption spectrometry by Perkin Elmer Optima 3100 XL.

### Example 5 - Preparation of a hyaluronate gel containing Na⁺₃[Ag⁺(MPS⁻²)₂]⁻³

To a 200 g amount of the aqueous solution of Na⁺₃[Ag⁺(MPS⁻²)₂]⁻³ disclosed in Example 4, 4, 97 g of distilled water and 3 g of sodium hyaluronate are added; the mixture is kept under gentle stirring for 12-16 h, until complete jellification. %Ag⁺ = 0.07%

### Example 6 - Preparation of a hyaluronate gel containing Na⁺₃[Ag⁺(MPS⁻²) ₂]⁻³ and sodium ascorbyl phosphate

To a 300 g amount of the aqueous solution of Na⁺₃[Ag⁺(MPS⁻²)₂]⁻³ disclosed in Example 4, 20g of sodium ascorbyl phosphate are added, followed by 76 g of distilled water and 4 g of sodium Hyaluronate. The mixture is kept under gentle stirring for 12-16 h, until complete jellification. %Ag⁺= 0.08%.

### Example 7 - Preparation of an aqueous solution containing Na⁺₃[Ag⁺(X⁻²)₂]⁻³

Reactants: NaX = 2-Mercapto-5-benzimidazole sulfonic acid, sodium salt bi-hydrate; CAS: 207511-11-3; MW = 288.28; Ag(NO₃), MW 169.87, CAS 7761-88-8.

A 1.9 g amount of NaX is dissolved in 898 g of water and 0.56 g of AgNO3, dissolved in 99.44 g of water, are added under stirring. The solution is kept under stirring for 30 min. The white solid silver complex is precipitated by addition of acetone and dried at 60°C.

Elemental analysis on the solid product gave the following results: calculated for Na₃AgS₄N₄O₆C₁₄H₈: C 26.55; N, 8.85; H, 1.27; Ag, 17.03. Found: C, 25.89; N 8.60; H 1. 32; Ag 16.90. Elemental analysis of carbon, nitrogen and hydrogen was performed with a LECO CHN analyzer. Silver was determined through plasma atomic absorption spectrometry by Perkin Elmer Optima 3100 XL.

As previously disclosed with reference to Na⁺₃[Ag⁺(MPA⁻²)₂]⁻³ and Na⁺₃[Ag⁺(MPS⁻²)₂]⁻³, also with Na⁺₃[Ag⁺(X⁻²)₂]⁻³ stable gel solutions in presence of sodium hyaluronate and sodium ascorbyl phosphate were prepared by using analogous procedures.

### Example 8 - Preparation of an aqueous solution containing Na⁺₃[Ag⁺(TS⁻²)₂]⁻³

Aqueous solutions of the complex were obtained for reaction of the TS²⁻ ligand, by adding sodium hydroxide with silver nitrate in 2:1 stoichiometric ratio and following procedures analogous to those disclosed in the previous Examples 1, 4 and 7. The solid complex was precipitated by addition of acetone and dried at 60°C.

Elemental analysis on the solid product gave the following results: calculated for Na₃AgS₂O₄C₁₄H₈ : C 34.94; H, 1.68; Ag, 22.42. Found: C, 34.26; H 1.78; Ag 21.76. Elemental analysis of carbon, nitrogen and hydrogen was performed with a LECO CHN analyzer. Silver was determined through plasma atomic absorption spectrometry by Perkin Elmer Optima 3100 XL.

### Example 9 - Preparation of salts formed by the silver complex with cationic surface-active agents

All the salts formed by the silver complex (according to the invention) with cationic surface-active agents have been prepared by mixing stoichiometric amounts of the silver complexes, dissolved in water, with aqueous solutions of the cationic surface-active agents in order to give the following neutral salts:

(OCT)₃[Ag⁺(MPA²⁻)₂]₂, (CHL)₃[Ag⁺(MPA²⁻)₂]₂, (BZ)₃[Ag⁺(MPA²⁻)2_{]}, (DDA)₃[Ag⁺(MPA²⁻)₂];

(OCT)₃[Ag⁺(MPS²⁻)₂]₂, (CHL)₃[Ag⁺(MPS²⁻)₂]₂, (BZ)₃[Ag⁺(MPS²⁻)₂], (DDA)₃[Ag⁺(MPS²⁻)₂];

(OCT)_{3[}Ag⁺(X²⁻)₂]₂, (CHL)₃[Ag⁺(X²⁻)₂]₂, (BZ)₃[Ag⁺(X²⁻)₂]; (DDA)₃[Ag⁺(X²⁻)₂];

(OCT)₃[Ag⁺(TS²⁻)₂]₂, (CHL)₃[Ag⁺(TS²⁻)₂]₂, (BZ)₃[Ag⁺(TS²⁻)₂], (DDA)₃[Ag⁺(TS²⁻)₂].

After mixing the two solutions, the precipitation of the neutral salts occurs immediately and all the solid matter can be easily isolated by vacuum filtration, washed with plenty of water, air dried and finally dried at 50°C in a ventilated oven.

Elemental analysis of carbon, nitrogen and hydrogen was performed with a LECO CHN analyzer. Silver was determined through plasma atomic absorption spectrometry by Perkin Elmer Optima 3100 XL. The elemental analysis of the carbon, nitrogen and hydrogen contents reported below are consistent with all formulations of the complex salts:
(OCT)₃[Ag⁺(MPA²⁻)₂]₂: calculated for Ag₂C₁₂₉N₁₆H₁₈₂S₈O₈ : C,60.59; H, 7.17; N, 8.76; Ag, 8.44; O, 5.01; S, 10.03. Found: C, 59.23; H, 7.66; N, 8.45.
(OCT)₃[Ag⁺(MPS²⁻)₂]₂: calculated for Ag₂C₁₁₇N₁₂H₁₈₆S₈O₁₂ : C, 57.95; H, 7.73; N, 6.93; Ag, 8.90; O, 7.92; S, 10. 58. Found: C, 56.78; H, 8.14; N, 6.12.
(CHL)₃[Ag⁺(MPA²⁻)₂]₂: calculated for Ag₂C₉₀N₃₄H₁₁₆S₈O₈Cₗ₆ : C, 44.03; H, 4.76; N, 19.40; Ag, 8.79; O, 3.91; S, 10.45; Cl, 8.66. Found: C, 43.12; H, 5.02; N, 18.87.
(CHL)₃[Ag⁺(MPS²⁻)₂]₂: calculated for Ag₂C₉₀N₃₄H₁₁₆S₈O₈Cₗ₆ : C, 44.03; H, 4.76; N, 19.40; Ag, 8.79; O, 3.91; S, 10.45; Cl, 8.66. Found: C, 43.12; H, 5.02; N, 18.87.
(DDA)₃[Ag⁺(MPA²⁻)₂]: calculated for AgC₇₈N₅H₁₅₄S₄O₄ : C,64.07; H, 10.62; N, 4.79; Ag, 7.36; O, 4.38; S, 8.72. Found: C, 63.66; H, 11.27; N, 4.34.
(DDA)₃[Ag⁺(MPS²⁻)_{2]}: calculated for AgC₇₂N₃H₁₅₆S₄O₆ : C,61.94; H, 11.26; N, 3.01; Ag, 7.73; O, 6.88; S, 9.19. Found: C, 60.32; H, 12.08; N, 2.86.

The elemental analysis of the benzalkonium-containing salts were in agreement with the prevalent presence of C12 alkyl chains with respect to the C14 and C16, which are known to be always present in the benzalkonium chloride product:
(BZ)₃[Ag⁺(MPA²⁻)₂]: calculated for AgC₆₉N₃H₁₁₂S₄O₄ : C,63.18; H, 8.61; N, 5.34; Found: C, 60.72; H, 9.74; N, 4.84.
(BZ)₃[Ag⁺(MPS²⁻)₂]: calculated for AgC₆₃N₃H₁₁₄S₄O₆ : C,60.74; H, 9.22; N, 3.37; Found: C, 58.83; H, 10.07; N, 2.98.

Elemental analysis on the salts containing the anionic complexes [Ag⁺(X²⁻)₂]³⁻ and [Ag⁺(TS²⁻)₂]³⁻ were not performed.

The solid salts were also obtained by mixing aqueous solutions of quaternary ammonium salts and of the silver complexes in a 3:1 molar ratio and by mixing aqueous solutions of chlorhexidine or octenidine with aqueous solutions of the silver complexes in a 3:2 molar ratio.

### Example 10 - Preparation of microbial mixtures for experimental use

The antimicrobial activity of aqueous solutions of the silver complexes and of corresponding gel formulations containing sodium hyaluronate and sodium ascorbyl phosphate were tested against mixtures of the microbial strains (purchased from Diagnostic International Distribution SpA) shown in Table 1 below:

**Table 1**

| Microbial species | Strain |
|---|---|
| *Pseudomonas aeruginosa* | ATCC 15442 |
| *Staphylococcus aureus* | ATCC 6538 |
| *Escherichia coli* | ATCC 10536 |
| *Enterococcus hirae* | ATCC 10541 |
| *Candida albicans* | ATCC 10231 |

Mixtures of the different strains of Table 1 with concentrations in the range 1.5×10⁹ - 5.5×10⁹ (CFUs) for each species were prepared.

The species of the microbial pool of Table 1 were selected since they are commonly found in skin wounds, as well as in the oral cavity when periodontal disease (periodontitis) occurs (Daniluk T. et al, Advances in Medical Sciences Vol 51, 2006, Suppl. 1.). In particular, human periodontitis is associated with a widely heterogeneous and complex subgingival microbiota, encompassing both Gram-positive and Gram-negative bacteria. Yeasts, especially *Candida albicans*, have been recovered in a large number from periodontal pockets in the oral cavity (Reynaud AH et al, J. Clin Periodontol, 2001; 28). Moreover, oral yeasts have been related with enamel and root caries (Järvensivu A et al, Oral. Dis, 2004; 10: 106-12) and aerobic bacteria are isolated more frequently from supragingival plaque compared to anaerobic bacteria. The microbial pool of Table 1 is therefore representative of the microbial population most frequently found in skin wounds and in the oral cavity.

### Example 11 - Tests on aqueous solution and gel containing the silver complex according to the invention.

The results disclosed in this Example are related to tests carried out on samples of aqueous solution and gel containing Na⁺₃[Ag⁺(MPA²⁻)₂]³⁻. However, analogous results have been obtained on similar solutions and gel containing Na⁺₃[Ag⁺(MPS²⁻)₂]³⁻.

Mixtures of the different strains of Table 1, having concentrations in the range of 1.5×10⁹ - 5.5×10⁹ CFU/ml for each species, were prepared and 100 µl aliquots of the mixtures were inoculated in Petri dishes containing Tryptone Soya Agar (TSA) as solid (agarized) culture medium. The inoculation was carried out according to a known and normed analytic method, namely by depositing the liquid aliquots on the surface of the agar through micropipette and distributing the liquid aliquots on the surface of the agar by using sterile glass beads. 100 µl samples of the silver complex Na⁺₃[Ag⁺(MPA²⁻)₂]³⁻ in aqueous solution or in gel were deposited inside the Petri dishes, in a substantially central zone of the agar. The Petri dishes were then incubated at 37° C for 24 h. After this time elapsed, the Petri dishes were examined, in order to evaluate the colonial proliferation and the inhibition halos (namely, portions of culture medium in which the microbial proliferation has been inhibited) surrounding the deposited silver complex.

As shown in Figure 1 and 2, clear inhibition halos surrounding the deposited silver complex were observed both for aqueous solution and gel, thus indicating that the samples containing the complexed silver ions are capable of inhibiting the growth of 1.5×10⁹ - 5.5×10⁹ CFU/ml of gram positive and gram negative bacteria, as well as of the fungal species *Candida albicans.*

Figure 1 shows a Petri dish in which an inhibition halo is observed, after deposition of a sample of 100 µl of aqueous solution containing Na⁺₃[Ag⁺(MPA²⁻)₂]³⁻(prepared according to Example 1) in presence of 100 µl of the microbial mixture having concentration 1.5×10⁹ - 5.5×10⁹ CFU/ml.

In Figure 2, the Petri dish on the left shows an inhibition halo observed after deposition a sample of 100 µl of gel containing Na⁺₃[Ag⁺(MPA²⁻)₂]³⁻ (prepared according to Example 3), in presence of 100 µl of the microbial mixture having concentration 1.5×10⁹ - 5.5×10⁹ CFU/ml, whilst the Petri dish on the right is a control Petri dish (no inhibition halo) after deposition of a control hyaluronate gel, containing the same amount of sodium-ascorbyl-phosphate disclosed in Example 3 (but not containing the silver complex according to the invention).

It should be noted that the gel formulations disclosed in Example 2, Example 3 Example 5 and Example 6 form a solid barrier once applied on the skin, thus favouring wound healing. In particular, evidence of this effect is disclosed in Example 14 (see below) with reference to the gel formulation of Example 6, hereinafter "liquid silver bandage".

### Example 12 - Inclusion tests on ethanol solutions of salts formed by the silver complex according to the invention with cationic surface-active agents

The results disclosed in this Example are related to tests carried out on ethanol solutions containing the salts formed by [Ag⁺(MPA²⁻)₂]³⁻ and cationic surface-active agents (test ethanol solutions). Analogous results were obtained by carrying out tests on similar solutions containing [Ag⁺(MPS²⁻)2_{]}³⁻ or [Ag⁺(X²⁻)₂]³⁻ and the same cationic surface-active agents.

Mixtures of the different strains of Table 1, having concentrations in the range of 1.5×10⁹ - 5.5×10⁹ CFU/ml for each species, were prepared and 100 µl aliquots of the mixtures were inoculated in Petri dishes and put in contact for 5 min with a dried sample of 100 µl of the test ethanol solutions containing the salts disclosed in Example 9. After pouring Tryptone Soya Agar in the Petri dishes and enabling the TSA to solidify, the Petri dishes were incubated at 37°C for 24 h. After this time elapsed, the Petri dishes were examined, in order to evaluate the colonial proliferation.

As shown in Figure 3 and 4, substantially no growth of microbial colonies is observed in the Petri dishes containing the salts formed by the anionic complex [Ag⁺(MPA²⁻)₂]³⁻ and the cationic surface-active agents.

Figure 3 shows: a control Petri dish (on the left), inoculated with a 100 µl aliquot of the microbial mixture in concentration 1.5×10⁹ - 5.5×10⁹ CFU/ml, and a Petri dish (on the right) inoculated with a 100 µl aliquot that was put in contact (5 min) with the salt (BZ)₃[Ag⁺(MPA²⁻)₂]. In the control Petri dish the growth of microbial colonies is evident, whilst no growth is observed in the Petri dish on the right.

Figure 4 shows: a first Petri dish (on the left) inoculated with a 100 µl aliquot that was put in contact (5 min) with the salt (DDA)₃[Ag⁺(MPA²⁻)₂] and a second Petri dish (on the right) inoculated with a 100 µl aliquot that was put in contact (5 min) with the salt (OCT)₃[Ag⁺(MPA²⁻)₂]₂. No growth of colonies is observed in both the Petri dishes.

Figure 5 shows: a first Petri dish (on the left) inoculated with a 100 µl aliquot that was put in contact (5 min) with the salt (BZ)₃[Ag⁺(MPA²⁻)₂] and a second Petri dish (on the right) inoculated with a 100 µl aliquot that was put in contact (5 min) with the salt(DDA)₃[Ag⁺(MPA²⁻)₂]. No growth of colonies is observed in both the Petri dishes.

### Example 13 - Tests on silica particles functionalized with the salts formed by the silver complex according to the invention with cationic surface-active agents

The results disclosed in this Example are related to tests carried out on silica particles, onto which the salts formed by [Ag⁺(MPA²⁻)₂]³⁻ and cationic surface-active agents have been adsorbed (functionalized silica particles). Analogous results were obtained by carrying out tests on silica particles functionalized with the salts formed by [Ag⁺(MPS²⁻)₂]³⁻ and the same cationic surface-active agents.

Mixtures of the different strains of Table 1, having concentrations in the range of 1.5×10⁹ - 5.5×10⁹ CFU/ml for each species, were prepared and 100 µl aliquots of the mixtures were inoculated in Petri dishes containing Tryptone Soya Agar. Samples of a powder, consisting of silica particles functionalized with the salts, were spread onto the agar surface inside the Petri dishes. The Petri dishes were then incubated at 37° C for 24 h. After this time elapsed, the Petri dishes were examined, in order to evaluate the inhibition halos surrounding the functionalized silica particles.

As shown in Figure 6 - 8, clear inhibition halos surrounding the deposited powder were observed in all cases, indicating that the functionalized silica particles are capable of inhibiting the growth of 1.5×10⁹ - 5.5×10⁹ CFU/ml of gram positive and gram negative bacteria, as well as of the fungal species *Candida albicans.*

Figure 6 shows a Petri dish containing silica particles functionalized with the salt (OCT)₃[Ag⁺(MPA²⁻)₂]₂, Figure 7 shows a Petri dish containing silica particles functionalized with the salt (CHL)₃[Ag⁺(MPA²⁻)₂]₂ and Figure 8 shows a Petri dish containing silica particles functionalized with the salt (BZ)₃[Ag⁺(MPA²⁻)₂].

### Example 14 - Topical treatment of a chronic skin lesion

Skin wounds, either acute or chronic and independently of the origin thereof, always undergo microbial contamination and a same situation occurs in mucosal and periodontal diseases. Microbial colonization of an acute skin or mucosal wound is a fast process, which induces an inflammatory response in the host. Inflammations is an initial, essential and time-limited step in the wound healing process. However, if the microbial burden is not controlled, which is often due to biofilm formation, inflammation persists over the required period. A persistent inflammation induces a high concentration of metalloproteases and neutrophil-derived esterase in the wound bed. These proteins destroy the extracellular matrix, so that the reconstruction of the damaged tissue is hampered and the healing process is halted in a chronic state. The control of the microbial burden in a skin or mucosal wound bed is therefore considered a pre-requisite to obtain wound healing. Moreover, to obtain a wound healing, a proper, moist environment, must be maintained throughout the entire treatment period.

The gel formulation of Example 3, or liquid silver bandage (hyaluronate gel containing Na⁺₃[Ag⁺(MPA⁻²)₂]⁻³and sodium ascorbyl phosphate), is particularly suitable for controlling the microbial burden and maintaining a moist environment in a skin or mucosal wound bed. The aforesaid liquid silver bandage exerts an effective anti-microbial activity, owing to the presence of ionic silver, and the application thereof can maintain an optimal moist environment in the wound bed, owing to the presence of hyaluronate. Once applied to a wound bed, the hyaluronate solidifies and forms a barrier that permits gas exchange but prevents micro-trauma and microbial contamination from the surrounding skin. Moreover, such a barrier is beneficial in terms of reducing the pain in the patients.

The liquid silver bandage can be applied to every type of skin and mucosal wound immediately after the wound bed has been cleaned properly. Depending on the type of wound - i.e. acute or chronic - the wound can be cleaned properly by simply rinsing with saline or by performing a surgical debridement.

By way of example, a procedure for the use of the liquid silver bandage is summarized below:
- Bleeding from the cleaned wound bed must have been ceased;
- The wound bed must be carefully dried with a sterile gauze;
- Once applied on a clean and dried wound bed, the liquid silver bandage solidifies rapidly;
- A secondary dressing can be applied, or not, depending on variable factors (e.g. the type of wound, the site thereof, the type of patient);
- Afterwards, the liquid silver bandage can be applied again as many times as needed (e.g. once daily in a non-exuding wound and more frequently in an exuding wound).

Figure 9 and Figure 10 respectively show a chronic skin wound before and after the application of the liquid bandage of Example 6 for a 3-weeks period. Figure 10 clearly and unambiguously shows that the skin wound evolved to a complete healing.

As a person skilled in the art will understand, variations on and/or additions to what has been disclosed above are possible. For example, although the previously disclosed compositions were prepared on laboratory scale, the person skilled in the art is able to provide preparation procedures that are suitable for a production on industrial scale.

## Claims

1. Anionic bi-coordinated silver (I) complex, having antimicrobial activity and the general formula:
[Ag⁺(L²⁻)₂]³⁻
wherein L²⁻is a ligand selected from the group consisting of: bivalent anionic form of 2-mercapto-4-methyl-5-thiazoleacetic acid; 3-mercapto-1-propansulfonate; 2-mercapto-5-benzimidazole sulfonate; and thiosalicylate.

2. Sodium salt of the anionic bi-coordinated silver (I) complex according to claim 1, having antimicrobial activity and the general formula:
Na⁺₃[Ag⁺(L²⁻)₂]³⁻

3. Aqueous solution of the sodium salt according to claim 2, having antimicrobial activity.

4. Hyaluronic acid composition containing the bi-coordinated silver (I) complex according to claim 1, for use as medicament having antimicrobial activity.

5. Hyaluronic acid composition containing the sodium salt according to claim 2, for use as medicament having antimicrobial activity.

6. Hyaluronic acid composition according to claim 4 or 5, further containing sodium ascorbyl phosphate.

7. Hyaluronic acid composition according to any one of claims 4 to 6, for use as a liquid bandage.

8. Hyaluronic acid composition according to any one of claims 4 to 7, wherein said hyaluronic acid is in the form of sodium hyaluronate.

9. Hyaluronic acid composition according to claim 8, wherein the percentage content of said sodium hyaluronate is comprised between 0.8-1.4%.

10. Salt of the anionic bi-coordinated silver (I) complex according to claim 1, having antimicrobial activity and the general formula:
(W⁺)₃[Ag⁺(L²⁻)₂]ₙ
wherein:
- W⁺ is a cationic surface-active agent, selected from the group consisting of octenidine, chlorhexidine, benzalkonium and didecyldimethylammonium;
- n is 2 when W⁺ is octenidine or chlorhexidine;
- n is 1 when W⁺ is benzalkonium or didecyldimethylammonium.

11. Alcoholic solution of the salt according to claim 10, having antimicrobial activity.

12. Alcoholic solution according to claim 11, wherein said alcohol is selected from the group consisting of methanol, ethanol, n-propanol, 2-propanol and 3-methoxy-3-methyl-1-butanol.

13. Dimethyl sulfoxide solution of the salt according to claim 10, having antimicrobial activity.

14. Antimicrobial solution for coating a solid surface, containing the salt according to claim 10, or the alcoholic solution according to claim 11 or 12, or the dimethyl sulfoxide solution according to claim 13.

15. Powder made of silica particles, wherein the salt according to claim 10, or the alcoholic solution according to claim 11 or 12, or the dimethyl sulfoxide solution according to claim 13 are adsorbed onto said particles, for use in an antimicrobial topical treatment.

## Patentansprüche

1. Anionischer bikoordinierter Silber (I)-Komplex mit antimikrobieller Wirkung und der folgenden allgemeinen Formel:
[Ag⁺(L²⁻)₂]³⁻
wobei L²⁻ ein Ligand ist, der aus der Gruppe ausgewählt ist, die aus Folgendem besteht: bivalenter anionischer Form von 2-Mercapto-4-Methyl-5-Thiazolessigsäure; 3-Mercapto-1-Propansulfonat; 2-Mercapto-5-Benzimidazolsulfonat; und Thiosalicylat.

2. Natriumsalz des anionischen bikoordinierten Silber (I)-Komplexes nach Anspruch 1 mit antimikrobieller Wirkung und der folgenden allgemeinen Formel:
Na⁺₃[Ag⁺(L²-)₂]³-

3. Wässrige Lösung des Natriumsalzes nach Anspruch 2 mit antimikrobieller Wirkung.

4. Hyaluronsäurezusammensetzung, die den bikoordinierten Silber (I)-Komplex nach Anspruch 1 enthält, zur Verwendung als Arzneimittel mit antimikrobieller Wirkung.

5. Hyaluronsäurezusammensetzung, die das Natriumsalz nach Anspruch 2 enthält, zur Verwendung als Arzneimittel mit antimikrobieller Wirkung.

6. Hyaluronsäurezusammensetzung nach Anspruch 4 oder 5, die ferner Natriumascorbylphosphat enthält.

7. Hyaluronsäurezusammensetzung nach einem der Ansprüche 4 bis 6 zur Verwendung als flüssiges Pflaster.

8. Hyaluronsäurezusammensetzung nach einem der Ansprüche 4 bis 7, wobei die besagte Hyaluronsäure in Form von Natriumhyaluronat vorliegt.

9. Hyaluronsäurezusammensetzung nach Anspruch 8, wobei der prozentuale Gehalt des besagten Natriumhyaluronats zwischen 0,8-1,4 % liegt.

10. Salz des anionischen bikoordinierten Silber (I)-Komplexes nach Anspruch 1 mit antimikrobieller Wirkung und der folgenden allgemeinen Formel:
(W⁺)₃[Ag⁺(L²⁻)₂]ₙ
wobei:
- W⁺ ein kationisches oberflächenaktives Mittel ist, das aus der Gruppe ausgewählt ist, die aus Octenidin, Chlorhexidin, Benzalkonium und Didecyldimethylammonium besteht;
- n 2 ist, wenn W⁺ Octenidin oder Chlorhexidin ist;
- n 1 ist, wenn W⁺ Benzalkonium oder Didecyldimethylammonium ist.

11. Alkoholische Lösung des Salzes nach Anspruch 10 mit antimikrobieller Wirkung.

12. Alkoholische Lösung nach Anspruch 11, wobei der besagte Alkohol aus der Gruppe ausgewählt ist, die aus Methanol, Ethanol, n-Propanol, 2-Propanol und 3-Methoxy-3-Methyl-1-Butanol besteht.

13. Dimethylsulfoxidlösung des Salzes nach Anspruch 10 mit antimikrobieller Wirkung.

14. Antimikrobielle Lösung zum Beschichten einer festen Oberfläche, die das Salz nach Anspruch 10 oder die alkoholische Lösung nach Anspruch 11 oder 12 oder die Dimethylsulfoxidlösung nach Anspruch 13 enthält.

15. Pulver aus Kieselsäurepartikeln, wobei das Salz nach Anspruch 10 oder die alkoholische Lösung nach Anspruch 11 oder 12 oder die Dimethylsulfoxidlösung nach Anspruch 13 auf den besagten Partikeln adsorbiert sind, zur Verwendung in einer antimikrobiellen topischen Behandlung.

## Revendications

1. Complexe d'argent (I) bicoordonné anionique, ayant une activité antimicrobienne et la formule générale :
[Ag⁺(L²-)₂]³⁻
où L²⁻ est un ligand sélectionné dans le groupe consistant en : forme anionique bivalente de l'acide 2-mercapto-4-méthyl-5-thiazoleacétique ; 3-mercapto-1-propansulfonate; 2-mercapto-5-benzimidazole sulfonate ; et thiosalicylate.

2. Sel de sodium du complexe d'argent (I) bicoordonné anionique selon la revendication 1, ayant une activité antimicrobienne et la formule générale :
Na⁺₃[Ag⁺(L²-)₂]³-

3. Solution aqueuse du sel de sodium selon la revendication 2, ayant une activité antimicrobienne.

4. Composition d'acide hyaluronique contenant le complexe d'argent (I) bicoordonné selon la revendication 1, pour une utilisation comme médicament ayant une activité antimicrobienne.

5. Composition d'acide hyaluronique contenant le sel de sodium selon la revendication 2, pour une utilisation comme médicament ayant une activité antimicrobienne.

6. Composition d'acide hyaluronique selon la revendication 4 ou 5, contenant en outre du phosphate d'ascorbyle de sodium.

7. Composition d'acide hyaluronique selon l'une quelconque des revendications 4 à 6, pour une utilisation comme un pansement liquide.

8. Composition d'acide hyaluronique selon l'une quelconque des revendications 4 à 7, où ledit acide hyaluronique est sous la forme d'hyaluronate de sodium.

9. Composition d'acide hyaluronique selon la revendication 8, où la teneur en pourcentage dudit hyaluronate de sodium est comprise entre 0,8 et 1,4 %.

10. Sel du complexe d'argent (I) bicoordonné anionique selon la revendication 1, ayant une activité antimicrobienne et la formule générale :
(W⁺)₃[Ag⁺(L²⁻)₂]ₙ
où :
- W⁺ est un agent tensioactif cationique, sélectionné dans le groupe consistant en octénidine, chlorhexidine, benzalkonium et didécyldiméthylammonium ;
- n est égal à 2 lorsque W⁺ est de l'octénidine ou de la chlorhexidine ;
- n est égal à 1 lorsque W⁺ est du benzalkonium ou du didécyldiméthylammonium.

11. Solution alcoolique du sel selon la revendication 10, ayant une activité antimicrobienne.

12. Solution alcoolique selon la revendication 11, où ledit alcool est sélectionné dans le groupe consistant en méthanol, éthanol, n-propanol, 2-propanol et 3-méthoxy-3-méthyl-1-butanol.

13. Solution de diméthylsulfoxyde du sel selon la revendication 10, ayant une activité antimicrobienne.

14. Solution antimicrobienne de revêtement d'une surface solide, contenant le sel selon la revendication 10, ou la solution alcoolique selon la revendication 11 ou 12, ou la solution de diméthylsulfoxyde selon la revendication 13.

15. Poudre faite de particules de silice, où le sel selon la revendication 10, ou la solution alcoolique selon la revendication 11 ou 12, ou la solution de diméthylsulfoxyde selon la revendication 13 sont adsorbés sur lesdites particules, pour une utilisation dans un traitement topique antimicrobien.
